# EUROPEAN PATENT SPECIFICATION

(11) **EP 1 362 236 B1**
(45) Date of publication and mention of the grant of the patent: **06.06.2012**
(21) Application number: 02700069.4
(22) Date of filing: 21.02.2002
(51) Int. Cl.: G01N 27/416

(54) **SENSOR FOR ANALYSIS OF MIXTURES BY GLOBAL SELECTIVITY AND ITS USE IN SENSOR SYSTEM**
SENSOR ZUR ANALYSE VON MISCHUNGEN DURCH GLOBALE SELEKTIVITÄT UND SEINE VERWENDUNG IN EINEM SENSORSYSTEM
CAPTEUR PERMETTANT L'ANALYSE DE MELANGES PAR LA SELECTIVITE GLOBALE ET SON UTILISATION DANS UN SYSTEME DE DETECTION

(30) Priority: 21.02.2001 BR 0103502; 30.01.2002 BR 0200409
(43) Date of publication of application: 19.11.2003
(73) Proprietor: Empresa Brasileira de Pesquisa Agropecuária - EMBRAPA, 3o Andar CEP-70770-901 Brasilia, DF (BR)
(72) Inventor: VENANCIO, Everaldo, Carlos, CEP-13560-470 Sao Carlos, SP (BR); NETO, Ladislau, Martin, CEP-13561-260 Sao Carlos, SP (BR); RIUL, Antonio, Junior, CEP-13560-181 Sao Carlos, SP (BR); FONSECA, Fernando, Josepetti, CEP-05406-100 Sao Paulo, SP (BR); CAPPARELLI MATTOSO, Luiz, Henrique, CEP-13564-100 Sao Carlos, SP (BR); MELLO, Sarita, Vera, CEP-82810-160 Capao da Imbuia, PR (BR); TAYLOR, David Martin, Inst. of Mol. & Biom. Elec., Bangor, Gwyendd LL57 1UT (GB)
(74) Representative: Grünecker, Kinkeldey, Stockmair & Schwanhäusser
(86) International application number: PCT/BR2002/000028
(87) International publication number: WO 2002/066970

(56) References cited:
- EP-A1- 0 821 228
- WO-A2-00/63682
- US-A- 5 334 351
- US-A- 5 698 089
- US-A- 5 766 934
- PATENT ABSTRACTS OF JAPAN vol. 17, no. 448 (P-1594) 17 August 1993 & JP 05 099 896 A (ANRITSU) 23 April 1993
- STUSSI E ET AL: "Chemoresistive conducting polymer-based odour sensors: Influence of thickness changes on their sensing properties", SENSORS AND ACTUATORS B, ELSEVIER SEQUOIA S.A., LAUSANNE, CH, vol. 43, no. 1-3, 1 September 1997 (1997-09-01), pages 180-185, XP004103396, ISSN: 0925-4005, DOI: DOI:10.1016/S0925-4005(97)00147-0
- PERSAUD K ET AL: "ANALYSIS OF DISCRIMINATION MECHANISMS IN THE MAMMALIAN OLFACTORY SYSTEM USING A MODEL NOSE", NATURE, NATURE PUBLISHING GROUP, LONDON, GB, vol. 299, no. 5881, 23 September 1982 (1982-09-23), pages 352-355, XP000569767, ISSN: 0028-0836, DOI: DOI:10.1038/299352A0
- GUADARRAMA A ET AL: "Array of sensors based on conducting polymers for the quality control of the aroma of the virgin olive oil", SENSORS AND ACTUATORS B, ELSEVIER SEQUOIA S.A., LAUSANNE, CH, vol. 69, no. 3, 25 October 2000 (2000-10-25), pages 276-282, XP004218225, ISSN: 0925-4005, DOI: DOI:10.1016/S0925-4005(00)00507-4
- PERSAUD K.C. ET AL: 'MEASUREMENT OF SENSORY QUALITY USING ELECTRONIC SENSING SYSTEMS' MEASUREMENT AND CONTROL, INSTITUTE OF MEASUREMENT AND CONTROL. LONDON, GB vol. 29, no. 1, 01 February 1996, pages 17 - 20, XP000557284 ISSN: 0020-2940
- PEARCE T C ET AL: "ELECTRONIC NOSE FOR MONITORING THE FLAVOUR OF BEERS", ANALYST, LONDON, GB, vol. 118, no. 4, 1 April 1993 (1993-04-01) , pages 371-377, XP000196878, DOI: DOI:10.1039/AN9931800371
- PERSAUD K.C. ET AL: 'Application of conducting polymer odour sensing arrays to malodour monitoring' 19961030 30 October 1996, pages 6/1 - 6/4, XP006509691
- PEARCE T.C.: 'Computational parallels between the biological olfactory pathway and its analogue The Electronic Nose : Part II. Sensor-based machine olfaction' BIOSYSTEMS, NORTH-HOLLAND, AMSTERDAM, NL vol. 41, 01 January 1997, pages 69 - 90, XP007916532 ISSN: 0303-2647

## Description

### Field of the Invention

The present invention refers to a sensory system formed by different sensor units composed by interdigitated electrodes or microelectrodes, some of which are coated with ultra-fine films of complexing polymer conductors and their mixtures with other complexing substances for the evaluation and detection of substances based on the effect of global selectivity. The measurement system is based on the analysis of the response patterns of an electrical signal, obtained by means of electrical measurement of alternating current (AC), of the sensor system immersed in the medium to be analysed. The sensor may be employed, amongst other uses, in the analysis of taste and for monitoring the quality of products intended for human consumption, especially beverages and foods in liquid form, and in the evaluation and detection of humic substances and other contaminants (organic or inorganic) in water from natural sources (rivers, lakes, ponds, etc.) with the purpose of monitoring the quality of the water and environmental conservation.

### Background of the Invention

The need to evaluate and detect components in mixtures has been the reason for developing increasingly sensitive and sophisticated instruments that allow the quality control of substances essential to man, such as water and air, and of consumer goods, such as foods, beverages and drugs. The sensors developed for these purposes are different in constitution, taking into consideration the type of substance to be detected and the quantity in which this substance is present in the mixture. However, when dealing with a set of characteristics, whether organoleptic or levels of contamination, the detection of the individual components is frequently of no interest, but rather the evaluation of the resulting combination, such as flavour, odour, the total effect of contamination (i.e. humic substances present in water for human consumption). This evaluation of the group is known as global selectivity effect.

The presence of organic material dissolved in water (mainly constituted of humic substances) is a factor that greatly affects the quality of the water because during treatment with chlorine there frequently occurs the formation of carcinogenic matter such as tri-halogenated compounds, especially tri-halomethane. The bad quality of the water caused by the presence of humic substances is largely due to the fact that the techniques for planting and administering the soil in many countries - including Brazil - are still done in an uncontrolled manner (see Resk, D.V.S. in: II Encontro Brasileiro sobre Substâncias Húmicas, p50, São Carlos, SP, 1997). In fact, the improper use of natural resources also leads to environmental degradation, either through the direct contamination of the soil and water by toxic products, or by the destruction of forests which has led to erosion and silting, amongst other consequences. Thus, it is very important to monitor the levels of such degradation. However, the devices known at present are of very limited use, principally because their use is restricted to laboratories, which frequently makes the analysis of material in places of difficult access impossible. Therefore, the development of cheaper and more easily handled devices that can be used directly in the field is desirable.

Thus, given the importance of maintaining low levels of humic substances - in view of the fact that they participate in various processes in sources of natural water (through the complexation of metals) and in the soil (in the retention of nutrients and pesticides) - the detection of these humic substances is fundamental not only to halt environmental degradation but also to serve as guidance in the treatment of the water to be consumed in urban areas. Therefore, the ideal situation would be to provide a sensor that detects the presence of humic substances - selecting them by their different categories according to their origin and through the recognition of their humic characteristics - and permits their classification and quantification, but with no concern as to the individual discrimination of their constituents or their precise chemical composition. More sophisticated instruments such as liquid and gas chromatography, elementary chemical analysis and various other physicochemical analysis techniques (see D. A. SKOOG and D. M. WEST, Principles of Instrumental Analysis. 4th Ed., Philadelphia: Saunders College Publishing (1992)) are at a disadvantage due to the complexity of analysis, the time needed for measurement and the high operational costs, apart from not permitting use in the field.

Just as in the case of the necessity of evaluating and detecting humic substances as a whole, the evaluation of an overall flavour is of fundamental importance for the quality control of beverages and food for human consumption and even veterinary use because the commercial viability of a product may depend on the acceptance of the flavour of the feed by the animal. In fact, the substances responsible for the different tastes (sour, salt, sweet, bitter and umami proteic flavour) are detected in a biological membrane found in special canals of the tongue, composed mainly of lipids and proteins. In other words, the human senses of smell and taste are not capable of distinguishing each one of the substances responsible for the flavour and odour separately, but rather identify basic patterns which are recognised by the brain through the concept of "global selectivity" described above. In this case, also, the only satisfactory sensor systems are those capable of evaluating cheaply and quickly the quality of different types of beverages, selecting them by different categories according to their flavour and thus allowing their quantification in a global manner, i.e. the type of coffee or wine.

The conventional chemical sensors base themselves on the recognition of specific chemical compounds, which renders them useless for recognising humic substances in solution or substances that compose a flavour due to the diversity of constituents present in a mixture.

In the case of the evaluation and detection of humic substances and organic matter present in soil and in aqueous systems, various sensors exist and are described in patent documents. The US 5.044.756 patent describes a sensor for the detection of organic matter, in real time, consisting in the use of a light source and detector that operate in dynamic mode, through the scan of the specific area of the soil in which the levels of organic matter are to be evaluated. The light signals obtained are processed in such a manner as to produce a specific mathematical analysis for each type of soil. Such a method is limited to solid samples, and therefore is not of any use for the detection of humic substances present in water. Document JP 05079988 presents a rapid method for detecting and quantifying humic acid, through the use of chemical luminescence, consisting in the stages of adding hydrogen peroxide and formaldehyde to the sample, adjusting the pH with alkali (sodium hydroxide) and, finally, measurement of the chemical luminescence (using the length of the maximum wave of luminescence) for the detection and quantification of humic acid, where the spectrum of luminescence is extracted and recorded in the form of an amplified electrical signal. Through the use of a photomultiplier and an amplifier the intensity of the luminescence obtained is compared with that which is obtained for known concentrations of humic acid (calibration curve). These types of sensors involve relatively high costs and require sophisticated equipment and treatments incompatible with the requirements of field tests.

Concerning the evaluation of taste, the known sensors are based on the imitation of biological detection seeking assimilation of the materials used with the natural membrane that allows the transport and identification of the basic standard of flavours. There are two principal types, with the first being based on the properties of lipids, which participate in the natural process of taste. The second determines the ions present in solution (i.e. mineral water and wines). The first type is generally formed by the dispersal of a lipid compound responsible for transducing the signal on a polymeric matrix, normally non-conducting, such as vinyl polychloride (see US 5.482.855, US 5.302.262, JP 05099896, JP 06174688, JP 10078406, JP 10267894, K. Toko, Measur. Sci. Technol. 9, p.1919 (1998); K. Toko, H. Akyiama, K. Chisaki, S. Ezaki, T. Iiyota, K. Yamafuji, Sensors and Materials, Vol. 9, N5, p.321 (1997), S. Ezaki, H. Kunihiro, Sensors and Materials, Vol. 11, N8, p.447 (1999)). This type of sensor has been used for the evaluation of both taste (it distinguishes the basic standards of sweet, salt, sour, bitter and different types of beers, coffees, mineral water, milk, wine, sake) and the quality of water and pollution in rivers (see H. Sakai, S. lyiama, K. Toko, Sensors and Actuators B, Vol. 66, p.251 (2000), patents US 5,482,855, US 5,302,262). However, various disadvantages related to the stability of the membranes, the weak adhesion to certain types of surfaces and the difficulty in miniaturising the system in consequence of the relatively large thickness of the polymeric layer, make known sensors unsuitable for differentiating flavours below the limits of biological detection. The second type of sensor works in the range of detecting ions in solutions through the use of selective membranes containing dispersed amorphous semi-conductors (i.e. PVC membranes containing these dispersed semi-conductors) and conventional electrodes (see C. DiNatale, A. Macagnano, F. Davide, A.D'Amico, A. Legin, Y. Vlasov, A. Rudnitskaya, B. Selenov, Sensors and Actuators B Vol. 44, p.423 (1997); A. Legin, A. Rudnitskaya, Y. Vlasov, C. DiNatale, E. Mazzone, A.'Damico, Electroanalysis, Vol. 11, N10, p.814 (1999); A. Legin, A. Rudnitskaya, Y. Vlasov, C. DiNatale, E. Mazzone, A.'Damico, Sensors and Actuators B, Vol. 65, p.232 (2000)), but these do not permit the detection of non-polar substances, such as coffee, for example, or those that do not form electrolytes, such as saccharose.

More recently, there have been proposals for sensors based on intrinsic electrical conductor polymers, or more simply, conducting polymers. It has been ascertained that apart from being electrically active responding to an electric stimulus, these polymers can also be used as sensitive and transducer materials in sensors for the detection of different substances.

Sensors employing conductive polymers are generally manufactured by forming films of these materials over a pre-determined configuration of metallic or semiconductor electrodes (see H.E. Endres, S. Drost, Sensors and Actuators B, Vol. 4, p.95-98 (1991), T.Hofmann, K. Schröde, J. Zacheja, J. Binder, Sensors and Actuators B, Vol. 37, p. 37-42 (1996)). The majority of the devices developed up to now are specific for gas systems and are either based on the use of a polymer conductor (see US 4.887.455, US 5.417.100, US 5.536.473) or, more commonly, composed by an array of various sensors involving a mixture of an organic polymer conductor (generally polypyrrol) with conventional polymers or other materials that differ in chemical composition from the first, such as polystyrene, poly(A-methyl styrene), poly(styrene-acrylonitryl), poly(styrene-maleic anhydride), poly(styrene- allyl alcohol), poly(N-vinylpyrrolidone, poly(4-phenolvinylic), poly(vinyl butyral), poly(vinyl acetate), poly(bis phenol carbonate). This difference in composition creates a variation in electrical resistance when a chemical substance is absorbed over the surface of these devices, producing a distinct response pattern for each sample analysed. Therefore, the majority of these sensors employ the same polymer conductor dispersed in other materials and distinguish both the pure vapours of volatile chemical substances as well as the binary mixtures of these vapours (see US 5.951.846, US 5.959.191, US 5.571.401) and also substances to be analysed present in different types of fluids (liquid or gas) (see US 6.010.616).

In fact, known sensors allow the detection of mixture components but do not normally characterise the group, with the exception of some types of aroma sensors (electronic noses). For such, a new concept of detection and quantification of specific classes of chemical compositions is being employed, which is the global selectivity concept (see K. Toko, Measurement Sci. Technol., v.9, p.1919, 1998; K. Toko, Sensors and Actuators B, v.64, p.205, 2000). This concept is based on the recognition of the response patterns that characterise different classes of chemical compounds through the use of an arrangement of different sensor units that form a sensory system. In this arrangement, each sensor unit is constituted of a material different from the other units and presents a distinct response for a specific group of chemical compounds. Thus, the system establishes a "finger print" of a specific compound.

However, the main difficulty in this technique is obtaining a sensitivity that surpasses the biological limit, allowing the differentiation of mixtures with very similar characteristics.

Another important point to be addressed is the fact that known sensory systems frequently deal with an electrical signal received from a sensor unit by means of components that use a direct electrical current (DC) (see F. Musio, M.C. Ferrara, Sensors and Actuators B, Vol. 41, p.97-103 (1997); M.E.H. Amrani, P.A. Paine, IEE Proc. Sci. Meas. Technol. Vol. 146, N2, p.95-101 (1999)). On the other hand, the use of measurements with alternating electrical current (AC) in sensors based on polymer conductors for the detection of gases is considered advantageous in view of the greater efficiency when compared with measurements with direct current. One of the disadvantages presented by the DC technique is the excessive time required for the systematic variation of the experimental parameters. AC modulation offers greater experimental flexibility. Another disadvantage of the DC technique is that in the systems based on polymer materials the electrical variations are very low, but whilst these can be measured through components working with AC (see WO 98/19153), they are difficult to detect in DC. Apart from this, AC measurements are non-destructive because they avoid the movement of ionised chemical types within the samples. This latter phenomenon can cause an irreversible alteration of its electrical properties. Thus, it is possible to reduce the number of sensors employed, and consequently, the size of the device, furthermore allowing measurements even more sensitive and precise than those obtained from direct current. This impedance spectroscopy in alternating current is well known and has been reported as more viable when compared with potentiometer measurements (see K. Toko, Measur. Sci. Technol. Vol.9, p.1919-1936 (1998); K. Toko, Biosensors and Bioelectronics, Vol. 13, p.701-709 (1998); A. Legin, A. Rudnitskaya, Y. Vlasov, C. DiNatale, E. Mazzone, A. D'Amico, Electroanalysis, Vol. 11, N10, p.814-820 (1999); K. Toko, T. Matsuno, K. Yamafuji, K. Hayashi, H. Ikezaki, K. Sato, R. Toukubo, Biosensors and Bioelectronics, Vol. 9, p.359-364 (1994); S. Iiyama, K. Toko, K. Yamafuji, Agric. Biol. Chem. Vol. 50, N11, p.2709-2714 (1986)) and voltametrics (see S. Iiyama, Y. Miyasaki, K. Hayashi, K. Toko, K. Yamafuji, H. Ikezaki, K. Sato, Sensors and Materials, Vol. 4, N1, p.21-27 (1992); I. Winquist, P. Wide, I. Lundstrom, Analytica Chimica Acta, Vol. 357, N1/2, p.21 (1997)) in gustative sensors, not only because of the simplicity of acquisition of the experimental data but also because of detecting small signal variations in devices based on polymers with non-electrolytic materials.

Thus, the necessity of increasing the sensibility and selectivity of known sensory systems can be evaluated by the market reaction concerning the poor performance of the sensors available.

### Summary of the Invention

The object of the present invention is to provide a means of detection and evaluation of mixtures, based on the concept of global selectivity, with enhanced sensitivity and selectivity.

A first embodiment refers to a sensor for the analysis of mixtures by global selectivity according to claim 1.

A second embodiment refers to a taste sensor for the analysis of mixtures by global selectivity according to claim 16.

A third embodiment refers to a sensor for the detection of humic substances for the analysis of liquid systems by global selectivity according to claim 29. A fourth embodiment concerns a sensorial system according to claim 39.

### Brief Description of the Drawings

Figure 1: Illustrates a type of interdigitated electrode employed in the present invention.
Figure 2: Shows the spectrums, in areas of visible UV, that prove the formation of poly(o-ethoxyanilin) complexes with humic substances.
Figure 3: Shows the differentiation of capacitance in relation to the frequency for the sensor units 2, 5 and 6 in a buffer solution with and without humic substance (15 mg/l).
Figure 4: Shows the variations of capacitance for the sensor units 2, 6, 8 and 9, for example, for phosphate buffer solutions containing 20 mg/l of humic substances.
Figure 5: Graphically represents the capacitance values obtained at 1 kHz for a phosphate buffer solution at pH 5.4 and for a buffer containing humic substance.
Figure 6: Graphically represents the overall results of the different responses of sensor unit 6 in the presence of different concentrations of humic substances.
Figure 7: Graphically represents a multivariate analysis of the principalcomponents ("Principal Component Analysis - PCA") for the data presented in figure 5(a) Shows the separation between the different humic substances (fulvic acid and humic acid) and (b) the different concentrations of these humic substances.
Figure 8: Presents the response of a group of 6 sensor units for taste for a solution of NaCl 5mM, given in terms of the capacitance of the microelectrode in relation to the frequency.
Figure 9: Graphically represents the characteristic response pattern for salt taste (NaCl and KCl), sour taste (HCl) and sweet taste of saccharose.
Figure 10: Graphically represents the characteristic response pattern for salt taste (NaCl) and sweet taste of saccharose.
Figure 11: Graphically represents the differentiated response pattern for the salt taste (NaCl and KCl), in different concentrations, including concentrations below the limit of detection by the human organism (5mM).
Figure 12: Graphically represents the capacitance response signals (at 400 Hz) in samples of mineral water.
Figure 13: Graphically represents the capacitance response signals (at 400 Hz) in samples of chamomile tea.
Figure 14: Graphically represents the capacitance response signals (at 400 Hz) in samples of coffee.

### Detailed Description of the Invention

To facilitate comprehension of the present invention the definitions of important terms are supplied below:
- Global selectivity - signifies the quantification of a combination or mixture of various types of substances that result in a compound effect, such as a synergistic effect or suppression effect amongst the substances. Flavour, odour, contamination by organic substances (such as humic substances) are some examples of this compound effect.Ultra-fine film deposited in monolayer - fine monolayers obtained by deposit in conditions that allow control of the monolayer thickness to a range measured in Ångström. In the present invention the thickness of the monolayer varies between 5 and 500 Å.
- Complexing polymer conductor - signifies the capacity of the substance to become inherently conductive of electrical signals, with such materials including organic polymers having poly-conjugated electron-X systems (such as double bonds, aromatic rings or heteroaromatic rings or triple bonds), i.e. polyaniline; polypyrrol; polyacetylene; polydiacetylene; polythiophene; poly(3,4-ethylenedioxithiophene)(PEDOT); polyisothionaphthalene; poly-heteroarylenevinyline, in which the heteroarylene group may be thiophene, furane or pyrrol; poly-p-phenylene, polyphthalocyanine and similars, as well their derivatives and their mixtures.
- Complexing substance - signifies a substance that has the property to interact chemically (reversible complexation) and/or physically (reversible adsorption) with the group of substances peculiar to a compound effect and possesses a structure that confers it electric transductor properties. Typical examples are the lipids that interact with the group of substances peculiar to the flavour and lignin that interact with the humic substances responsible for the contamination of water and soil by organic matter.
- Substances peculiar to the mixture - signifies the group of substances responsible for a specific compound effect, such as flavour, odour and the contamination by organic matter. Examples are humic substances (such as humic acid and fulvic acid), the substances responsible for flavour (sweet, salt, bitter and sour) or odour (gases and vapours).

One of the principal characteristics of the present invention is the use of ultra-fine films of various complexing systems based both on complexing substances and on complexing polymer conductors, as well as a mixture of both, so as to recognise flavour and contamination by organic matter, particularly by the analysis of liquid systems. The necessity of working with a small film thickness results from the knowledge that the sensitivity of sensor devices diminishes considerably with the thickness of the polymeric film (see E. Stussi, R. Stella, D. De Rossi, Sensors and Actuators B, Vol. 43, N1, p.180 (1997), WO 98/19153).

It is well known that conductive polymer have the property of presenting values of electrical conductivity that vary in range from isolator to conductor, including all the range of semi-conductors. This versatility confers properties to this class of materials that render them apt not only to substitute the conventional inorganic semi-conductors in various electronic devices - such as transistors, capacitors and diodes - but also to be employed as sensitive and transducer material in sensors for the detection of different substances, because being electrically active they respond to electric stimuli.

Conductive polymers appropriate for use in this present invention are described in many references such as the patents US 5.494.609, US 5.368.717, US 5.356.660, US 5.290.483, US 4.877.646 and US 4.803.096. The polymers preferred are mentioned in the definition for complexing polymer conductors, with polypyrrole and polyanilin and their derivatives being preferential.

In the present invention, the conductive polymers are also used combined with complexing substances to form the ultra-fine monolayer films.

In the case of the evaluation and detection of contamination of water or soil by organic matter, the preferred complexing substances can be cited as sulphonated lignin, complexing organic acids (camphosulphonic acid, toluenesulphonic acid and similar compounds with complexing properties) and complexes containing metallic ions (copper, iron, aluminium etc.), both because of recognising the patterns of humic substances but also due to these sensors being employed in agriculture, in environmental control and in the treatment of water for domestic and industrial consumption, amongst others. It has recently been found that the use of substances with structures similar to humic substances - such as sulphonated lignin - facilitate the transducing of an electrical signal generated in the detection of humic substances in mixtures, and is, therefore, important for the increase of the sensitivity of the sensors for measuring the contamination levels of water and soil, for example.

When the purpose is the evaluation of flavour, the appropriate complexing substances are those that interact with the substances peculiar to the group characteristic of each taste (sweet, bitter, salt and sour). Those mainly preferred are the lipid compounds, such as stearic acid, caproic acid and caprylic acid, as well as other materials such as metallic complexes and enzymes (lysozime etc.).

Thus, the sensors of the present invention are based on the conjugation of the properties of the complexing polymer conductors with complexing substances, which allows a significant increment in the response pattern of sensor devices due to the intrinsic properties of these materials, therefore increasing the sensitivity and selectivity - basic and essential characteristics for the manufacture of efficient sensor devices - and also removing the effects of interfering reactions. In fact, the complexing polymer conductor serves both as a sensitive material for the substance of interest as well as a transducer of the electric signal generated by this stimulus; it operates at room temperature, which is not the case with conventional systems based on semi-conductors such as tin dioxide; it is easy to manufacture, being able to be prepared in the form of polymeric films by standard techniques such as the self-assembly technique and the Langmuir-Blodgett technique. The polymeric membrane may also serve as a support matrix for immobilising specific molecules of interest, such as enzymes, complexing agents and metals with specific catalytic properties.

The sensor units of the invention are formed through the deposit of ultra-fine films on interdigitated microelectrodes. Various deposit techniques can be employed. Amongst these, the self-assembly technique and the Langmuir-Blodgett (LB) technique are the preferred ones.

The self-assembly technique has the advantages of being easily executed and not requiring sophisticated equipment. However, despite its simplicity this method produces ultra-fine films of controlled thickness that may be applied to different substrates as monolayers or bilayers (see W.B. Stockton and M.F. Rubner, Macromolecules, v.30, p.2717, 1997; Cheung, W.B. Stockton and M.F. Rubner, Macromolecules, v.30, p.2717, 1997; K. Ariga, Y. Lvov and T. Kunitake, J. Am. Chem. Soc., v.31, p.4309 1998; L.G. Paterno, L.H.C. Mattoso and O.N. Oliveira Jr. Quimica Nova Vol. 24, n.2, p.228-235 2001). The methodology employed in this technique consists basically in the immersion of a solid substrate - chemically modified - in a solution containing polyelectrolytes (polycations and/or polyanions). The immersions occurning alternately in the respective solutions of polyelectrolytes lead to the forming of a self-mounted film, which allow the making of bilayers (polycation/polyanion) or of monolayers from one of the polyelectrolytes. The forming of self-assembled ultra-fine films is done over interdigitated electrodes or microelectrodes used as solid substrate. The use of ultra-pure water is vital to the process. The immersion times of the unit formed by the solid substrate and the recently formed layers in the solutions containing the polyelectrolytes may vary from 1 to 30 minutes. The preferred times are 1, 3 and 10 minutes.

The Langmuir-Blodgett (LB) technique is also adequate for use with the present invention, being very well known to specialists (see G.G. Roberts, in "Langmuir-Blodgett Films", Plenum Press Pub., New York (1990)). Briefly, this technique consists in spreading organic substances previously dissolved in volatile organic solvents, such as chloroform, over a liquid subphase using a microsyringe or micropipette. The subphase is normally ultra-pure water. The organic solvent evaporates a few minutes after this spreading process, leaving only a monolayer of the material to be deposited, which covers the whole extension of the surface of the water. In this stage the films are called Langmuir films. Through the use of special equipment, called a Langmuir trough, the useful area occupied by these molecules over the liquid subphase may be reduced by means of compression of this monolayer with a pair of movable barriers. The successive immersions and removals of a solid substrate, which may be an interdigitated electrode, into the liquid subphase allows the transfer of these films from the surface of the water to the surface of the solid substrate. It is the control of this process that defines the thickness and build-up of the ultra-fine films (called Langmuir-Blodgett films). These are therefore made and controlled at molecular level. Compression speeds frequently employed are in the range of 1 to 10 mm/min, whilst the characteristic depositing speed of this technique occurs between 0.5 and 10 mm/min.

It is important to highlight that the thickness of the monolayer should be small and characteristically between 5 and 500 Å. Preferentially, the ultra-fine film deposited by monolayer is constituted of 1 to 20 layers, with the film having a maximum of 500 Å. But, preferentially, the film consists of around 10 layers, each with a thickness of around 20 Å.

The description of other techniques used to deposit polymeric films, such as the coating of surfaces by spreading the material to be deposited in the form of a solution over a solid substrate that rotates in determined angular frequencies, where the thickness of the film can be controlled by adjusting the rotation speed of the substrate (called spin coating), electropolymerisation, coating the surfaces of a solid substrate by spreading the material in the form of a solution which, after evaporation of the solvent leaves a coating of the material over the substrate (called casting) and others may be encountered in T. Skotheim, R. Elsenbaumer, J.R. Reynolds, "Handbook of Conducting Polymers", Marcel Dekker, New York (1998).

The electrodes or microelectrodes used as solid substrate in the preparation of the sensors for the present invention are made of metal, preferably gold, platinum, copper, aluminium or other conductive material - with gold being preferred - deposited over glass in the form of interdigits and having varying in number, geometry and size in accordance with the specifications of the components for the sensors and their applications.

The concept of associating sensor units of different characteristics to evaluate the properties of the group is the basis of the sensor assembly of the present invention. Thus, the combination of sensor units constituted of interdigitated metallic electrodes or microelectrodes without any coating with sensory units including interdigitated metallic electrodes and microelectrodes coated with ultra-fine films of complexing conductive polymers, complexing substances and their mixtures permits the differentiation between mixtures even when they possess very similar characteristics. This occurs because each sensor unit produces an electrical response pattern distinct from the others and the analysis of the overall response furnishes a "finger print" of the liquid being analysed. As an example, for the detection and evaluation of flavour it would be possible to combine a sensor unit constituted of non-coated interdigitated metallic electrodes or microelectrodes with sensor units including: (a) interdigitated metallic electrodes or microelectrodes coated with ultra-fine film of complexing substances, i.e. lipid substance; (b) interdigitated metallic electrodes or microelectrodes coated with ultra-fine film of complexing conductive polymer, i.e. polyanilin or polypyrrole and (c) interdigitated metallic electrodes or microelectrodes coated with ultra-fine film of complexing conductive polymer in combination with complexing substance. However, when using the sensor array of the invention for detecting contamination of water by organic matter, it would be possible to combine a sensor unit constituted of non-coated interdigitated metallic electrodes or microelectrodes with sensor units including: (a) interdigitated metallic electrodes or microelectrodes coated with ultra-fine film of complexing substances, i.e. sulphonated lignin; (b) interdigitated metallic electrodes or microelectrodes coated with ultra-fine film of complexing conductive polymer, i.e. polyanilin (poly(o-ethoxyanilin)) or polypyrrole and (c) interdigitated metallic electrodes or microelectrodes coated with ultra-fine film of complexing conductive polymer in combination with complexing substance.

The following examples aim to show the preferred embodiments of the invention. It will be obvious to specialists in the field that the procedures described in the examples represent means of executing the invention and, therefore, any modification of the conditions, stages or materials used that maintain the essential characteristics and remain within the working limits of the method of detection described, are included as part of the present invention.

### EXAMPLES

### Example 1: Deposit of the films on the interdigitated electrodes or microelectrodes

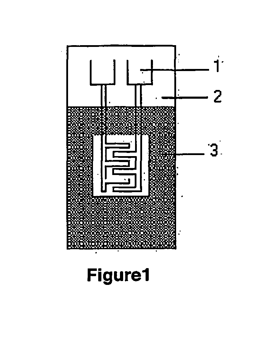

Gold electrodes, 1mm wide, with a spacing of 1mm and 70 nm of metal layer thickness and possessing appropriate configurations to work in the range of 2 to 100 digits are mounted on glass. As mentioned above, other dimensions and configurations are possible depending on the purpose of the sensor. For example, the electrodes may measure 10 µm in width, have a spacing of 10 µm and a metal layer thickness of 0.1 µm and be mounted on glass in 25 pairs as shown in Figure 1, where (1) represents the gold electrode, (2) represents the glass base and the shaded area (3) represents a protective coating of a photo-sensitive material that only exposes the useful area of the electrode.

### (A) Deposit by the self-assembly technique:

The electrodes where alternately immersed in an aqueous solution of hydrochloric acid at pH 5.0, containing the complexing materials - polyanion (sulphonated lignin) and polycation (poly(o-ethoxyanilin))- in concentrations ranging from 1x10⁻⁵ to 1x10⁻² mol 1⁻¹, with the best concentration generally being of 1x10⁻³ mol 1⁻¹, until obtaining a self-mounted ultra-fine film having from 1 to 20 alternating monolayers, generally 10 monolayers with a thickness of around 20 Å each, resulting in self-mounted ultra-fine films with a maximum thickness of 500 Å. All these solutions were prepared with ultra-pure water obtained from a Milli-Q^{®} system from MilliPore. The immersion times were of 3 minutes in both solutions containing the polyanion and polycation, respectively. The electrodes having a layer of the self-mounted films of poly(o-ethoxyanilin), of poly(o-ethoxyanilin) + sulphonated lignin + hydrochloric acid, of poly(o-ethoxyanilin) + sulphonated lignin + camphosulphonic acid, of poly(o-ethoxyanilin) + sulphonated lignin + toluenesulphonic acid, were prepared with different immersion times of 1, 3 and 10 minutes, in aqueous solutions of hydrochloric acid at pH 3.0 or pH 5.0 containing poly(o-ethoxyanilin), or poly(o-ethoxyanilin) + sulphonated lignin + hydrochloric acid, or poly(o-ethoxyanilin) + sulphonated lignin + camphosulphonic acid, or poly(o-ethoxyanilin) + sulphonated lignin + toluenesulphonic acid, respectively. The sensor units obtained in this manner were firstly washed by a process of immersion in an aqueous solution of hydrochloric acid at pH 3.0 or pH 5.0, in accordance with the respective pH values of the solutions containing the complexing materials which were used in the preparation of the sensor units, during 3 minutes. Finally, the sensor units were washed by a process of immersion in ultra-pure water obtained from a Milli-Q^{®} system from MilliPore, for 20 minutes.

### (B) Deposit by the LB technique:

The substance to form the film (complexing conductive polymer, complexing substance or a mixture of both) previously dissolved in volatile organic solvents, with chloroform being used in this case, is spread over a liquid subphase (ultra-pure water) in a Langmuir trough, using a microsyringe or micropipette. The organic solvent evaporates within a few minutes after this process of spreading, forming a monolayer of the material to be deposited which covers the whole extension of the surface of the water. The useful area occupied by the molecules of the film forming substance may be reduced by means of compressing this monolayer with a pair of movable barriers. A low compression speed was used (in the range of 1 to 10 mm/min), which improves the transfer of some conductive polymers to the surface of solid electrodes. The deposit speed was in the range of 0.5 to 10 mm/min.

### Example 2: Obtaining sensor units for the evaluation of contamination levels of water based on the analysis of the humic substances present in the sample.

Sensor units were prepared in accordance with the techniques (self-assembly and LB) described in Example 1 above, with 5 pure monolayers of an oligomer of polyanilin (16-mer) and polypyrrole (Ppy) being deposited on the electrodes using the Langmuir-Blodgett technique and self-assembled films of poly(o-ethoxyanilin) and sulphonated lignin using the self-assembly technique. These films only covered the exposed useful area of the interdigitated electrodes. Metallic wires are soldered to the upper squares (also of gold), which connect the device to a frequency response analyser.

Table 1 shows the prepared sensor units, which can integrate a sensor for humic substances.

**Table 1 - Sensor units for an electronic tongue based on conductive polymers and complexing substances.**

| Sensory Unit | Type of film deposited on the electrodes |
|---|---|
| 1 | None (interdigitated electrode without polymer coating) |
| 2 | POEA (poly(o-ethoxyanilin) film, obtained by self-assembly with an immersion time of 1 minute) |
| 3 | POEA (poly(o-ethoxyanilin) film, obtained by self-assembly with an immersion time of 3 minutes) |
| 4 | POEA (poly(o-ethoxyanilin) film, obtained by self-assembly with an immersion time of 10 minutes) |
| 5 | POEA+SL+HCl (poly(*o*-ethoxyanilin) + sulphonated lignin + hydrochloric acid film, obtained by self-assembly from a solution containing a complex of poly(o-ethoxyanilin) + sulphonated lignin + hydrochloric acid) |
| 6 | POEA+SL+CSA (poly(*o*-ethoxyanilin) + sulphonated lignin + camphosulphonic acid film, obtained by self-assembly from a solution containing a complex of poly(o-ethoxyanilin) + sulphonated lignin + camphosulphonic acid. |
| 7 | POEA+SL+TSA (poly(*o*-ethoxyanilin) + sulphonated lignin + toluenesulphonic acid film, obtained by self-assembly from a solution containing a complex of poly(o-ethoxyanilin) + sulphonated lignin + toluenesulphonic acid. |
| 8 | POEA/SL (poly(o-ethoxyanilin) + sulphonated lignin self-mounted film, with 5 bilayers) |
| 9 | Oligomer 16-mer (PANI)(oligomer of polyanilin, obtained using the Langmuir-Blodgett technique LB, with 15 layers) |

### Example 3: Proof of the complexing properties of conductive polymers employing the sensor units of the present invention.

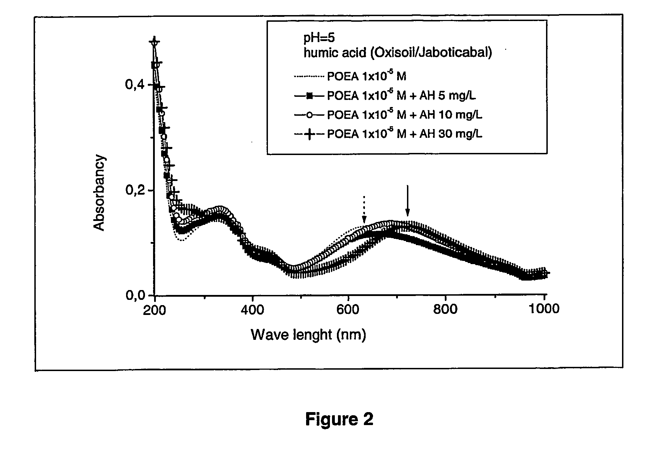

The interaction existing between the humic substances and the conductive polymers were verified using the spectroscopy technique in the ultra-violet and Visible regions (UV-Vis), between 190 and 1000 nm. Figure 2 shows the spectrum in the UV-Vis region that ascertain the formation of poly(o-ethoxyanilin) complexes with the humic substances.

In this system the analysis of the humic substances is undertaken in an aqueous solution at pH 5.0 (adjusted with hydrochloric acid). Such a system may also be employed as an optical sensor for humic substances.

Different solutions were prepared for the analysis of the humic substances: (1) aqueous solution of HCl at pH 5.0 (reference 1); (2) aqueous solution of HCl at pH 5.0, 1.0x10⁻⁵ and 5.0x10⁻⁵ mol/L of POEA, respectively; (3) aqueous solution of HCl at pH 5.0, 1.0x10⁻⁵ and 5.0x10⁻⁵ mol/L of POEA and different concentrations of humic substances, 5, 10 and 30 mg/l, respectively. The same set of solutions was prepared substituting the hydrochloric acid with a phosphate buffer at pH 5.4. Three different humic substances were analysed: humic acid obtained commercially from the Aldrich company, humic acid extracted from a purple latosoil from the region of Jaboticabal/SP (Brazil) and fulvic acid extracted from a distrophic oxysoilfrom the region of Campinas/SP (Brazil).

The operation of the sensory system was verified by obtaining the spectrums of absorption in the ultra-violet and visible regions, between 190 and 1000 nm, for the solutions 2 and 3 prepared exactly as described above. The standard solution used to acquire the absorption spectrums was an aqueous solution of hydrochloric acid (for the sensory system operating at pH 5.0) and phosphate buffer (for the sensor unit operating at pH 5.4). The spectrums obtained for the solutions 2 and 3 were then compared, with the movement of the polaronic band of the polymer present in the solution revealing the formation of a complex between the conductive polymer and the humic substance, thus allowing the detection of the latter.

In Figure 2 the results obtained with the sensory system operating at pH 5.0 detected the presence of humic acid from Aldrich in three different concentrations, 5, 10 and 30 mg/l. The same results were obtained in the presence of other humic substances, humic acid (HA) commercial (ALDRICH) and fulvic acid (FA), demonstrating the formation of complexes of POEA with HA (ALDRICH) and POEA with FA, respectively. These results clearly show that there is the formation of a complex between the POEA and the humic substances, demonstrating the importance of the use of this property (i.e. the formation of a complex causes alterations in the electrical properties of the POEA films) in monitoring the presence of humic substances in water.

### Example 4: Analysis of humic substances employing the sensory system of the present invention.

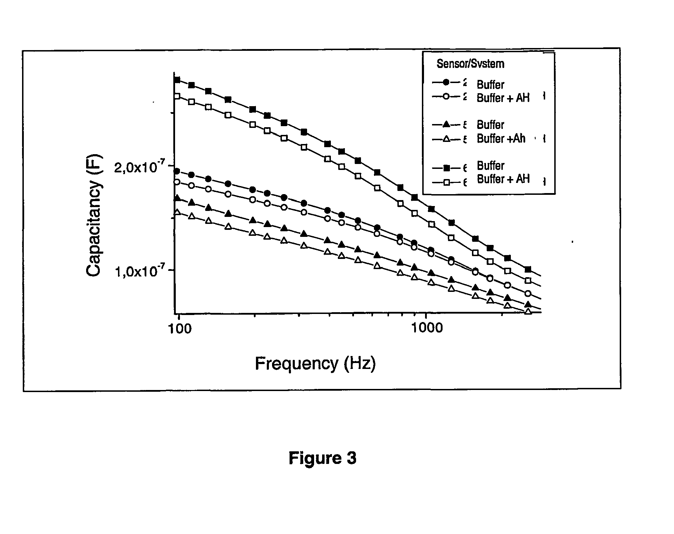

The sensor units obtained in Example 2 were assembled as a sensory system which also includes means of receiving electrical signals - through the measurement of alternating current - emitted by the sensor units in contact with the material to be analysed (humic substance) and means of processing the measurements for the analysis of a response pattern for the array of the different sensor units contained on Table 1 (see Example 2). The device formed in this manner was employed to analyse humic substances of different origins (regions). Figures 3 to 6 show the results obtained.

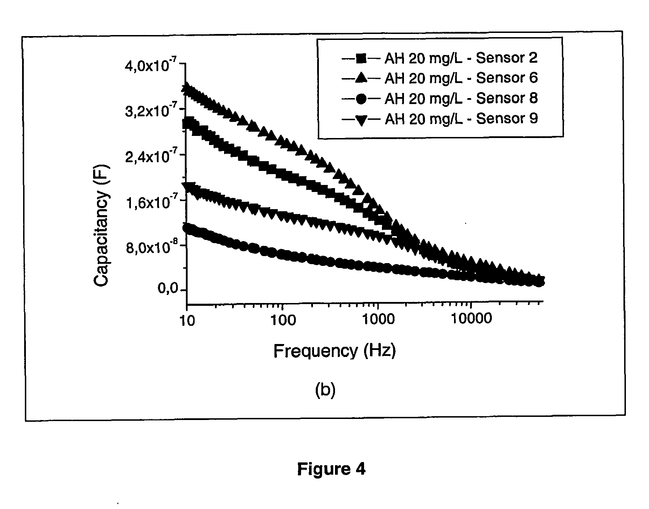

Three different humic substances were analysed: two samples of humic acid and one of fulvic acid, extracted from soils of different regions. The solutions of these samples were prepared with ultra-pure water (obtained from a Milli-Q^{®} system from MilliPore). The electrical response was obtained through an impedance analyser (mentioned earlier). This permits the sensory system to obtain a response pattern furnished in terms of capacitance of the microelectrode in function of frequency, which is distinct and characteristic for each type of humic substance analysed. Figures 3 and 4 show these results. As can be verified, the response obtained for each sensor unit is proportional to the quantity of humic substance present in the solution, demonstrating the possibility of a quantitative analysis. Figure 3 shows the differentiation of capacitance in function of the frequency for the sensor units 2, 5 and 6 immersed in buffer solutions with and without humic substances (15 mg/l, humic acid (ALDRICH)). This figure clearly illustrates the differentiation between a solution with and without the humic substance in a large interval of frequencies. Figure 4 shows the variation of capacitance for the sensor units 2, 6, 8 and 9, immersed in phosphate buffer solutions containing 20 mg/l of humic substance, (humic acid (ALDRICH)). It can be observed in this figure that the sensor units present different capacitance values for the same solution containing the humic substance. These responses highlight the selectivity, which allows the different humic substances to be distinguished because the sensor units present specific responses for the different solutions containing the humic substances.

The analysis of humic substances can also be done by selecting only one frequency, optimised for a range with a more selective response for the substance of interest being analysed.

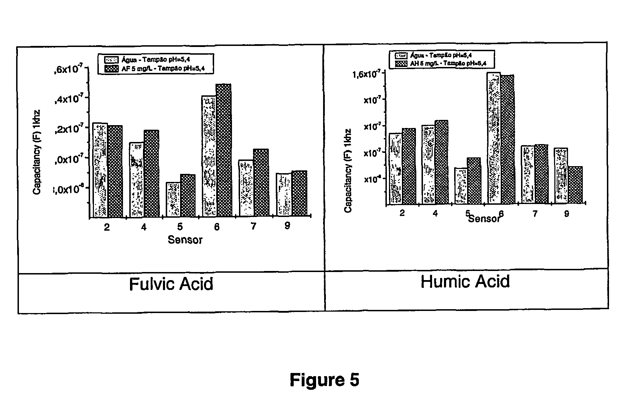

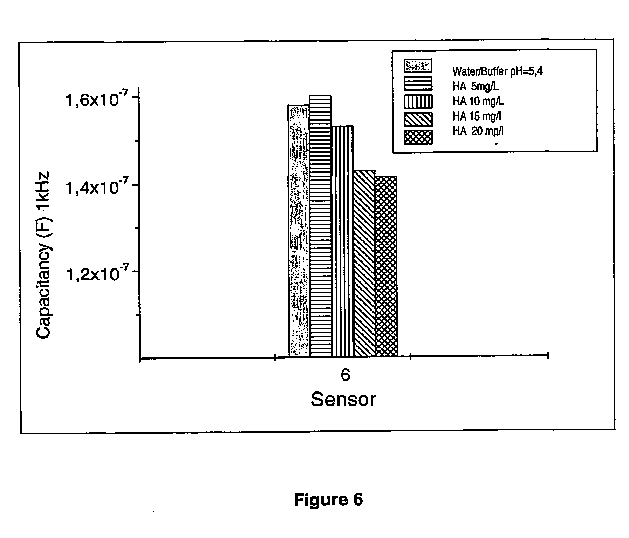

Figures 5 and 6 show the capacitance response for an assembly of 9 sensor units (as shown in Table 1), for the analysis of humic substances at a fixed frequency of 1 kHz. The distinction of signal amongst the various sensor units - illustrated in Figure 5 - was systematic and characteristic for each one of the solutions analysed and is also reproducible. Figure 5 presents the values of capacitance obtained at 1 kHz for a phosphate buffer solution with pH 5.4 and for the same buffer solution containing humic solution. The values presented for fulvic acid (distrophic oxysoil/Campinas/SP) and humic acid (ALDRICH) are the "finger prints" obtained by the use of different sensor units, thus distinguishing the two different solutions. Figure 6 presents an overall picture of the different responses of sensor unit 6 in the presence of different concentrations of humic substances, (humic acid (ALDRICH)). The concentration of humic substances vary from 5 to 20 mg/L. The results presented in Figure 6 show that the sensory system can differentiate the various concentrations of humic substances and also show its use in the quantification of the presence of humic substances. Furthermore, for each concentration a different response pattern can be observed, which allows the different concentrations of each humic substance to be distinguished.

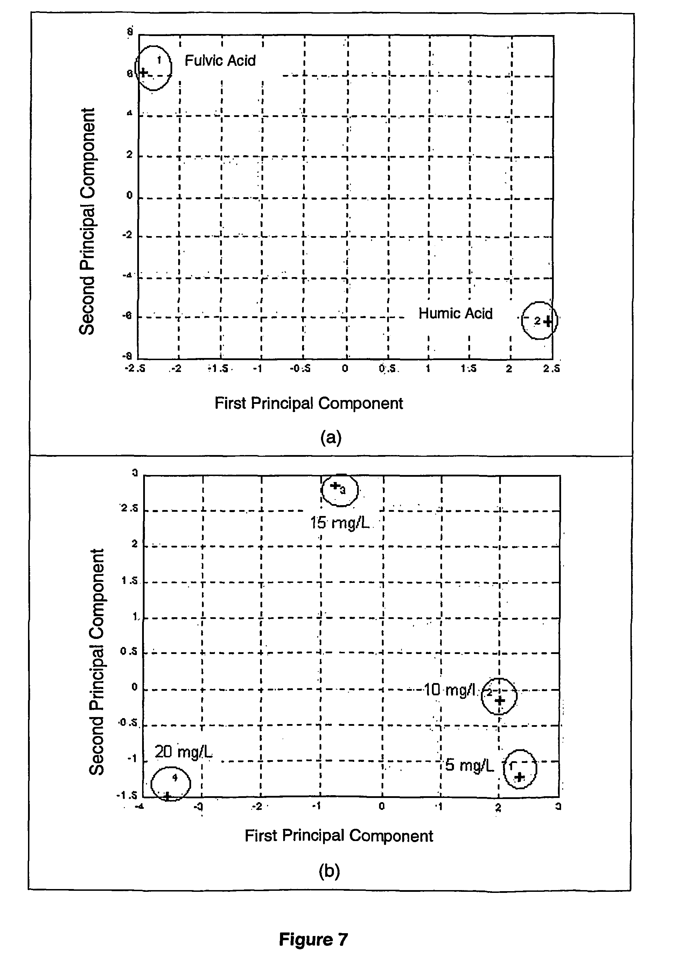

Figure 7 shows a multivariate analysis of the principal components ("Principal Component Analysis - PCA") for the data presented by Figure 5. This system clearly illustrates the separation of the different humic substances (fulvic acid and humic acid - see Figure 7a) and the different concentrations of these humic substances (commercial humic acid (ALDRICH) - see Figure 7b).

Equally important as the quantitative analysis of merely one substance is the qualitative analysis among various substances, which the present sensor can undertake in a very satisfactory manner. In this case, the device must be formed from different sensor units. The number of and the composition of the film of each sensor unit must be optimised in accordance with the characteristics of the humic substances to be analysed.

The separation among the different humic substances may be done without any problem through the measurement method of the present invention and the quantification can easily be done using appropriate software such as those existing for the analysis of neural networks (see J.W.Gardner, E.L. Hines, H.C. Tang, Sensors and Actuators B, Vol. 9, p.9-15 (1992)), because the variations in the concentrations of the substances imply in variations in the signal responses of the sensors. Also, there occurs a differentiated response sensibility for each sensor unit in accordance with the humic substance analysed. As an example of this, it can be seen that sensors 4, 5, 6, 7 and 8 show greater sensitivity to fulvic acid.

### Example 5: Obtaining sensor units for evaluating taste.

Sensory units were prepared in accordance with the techniques (self-assembly and LB) described in Example 1 above, with 5 pure monolayers of an oligomer of polyanilin (16-mer) and polypyrrole (Ppy) and also mixed films of these polymers with stearic acid being deposited on the electrodes using the Langmuir-Blodgett technique. These films only covered the exposed useful area of the interdigitated electrodes. Metallic wires are soldered to the upper squares (also of gold) which connect the device to a measurement device.

Table 2 shows the prepared sensor units that can integrate an *electronic tongue.*

**Table 2 - Sensor units for an electronic tongue based on conductive polymers and complexing substances.**

| Sensory Unit | Type of film deposited on the electrodes |
|---|---|
| 1 | None (interdigitated electrode without polymer coating) |
| 2 | Stearic acid |
| 3 | Polyanilin (oligomer 16-mer) |
| 4 | Ppy (Polypyrrole) |
| 5 | Polyanilin (oligomer 16-mer)/stearic acid |
| 6 | Ppy (polypyrrole)/stearic acid |

### Example 6: Analysis of taste (sweet, salt, sour and bitter) using the sensory system of the present invention.

The sensor units obtained in Example 5 were assembled as a sensory system which also includes means of receiving electrical signals - through the measurement of alternating current - emitted by the sensor units in contact with the beverage to be analysed and means of processing the measurements for the analysis of a response pattern of the array of the different sensor units contained on Table 2. The device formed in this manner was employed to analyse different flavours.

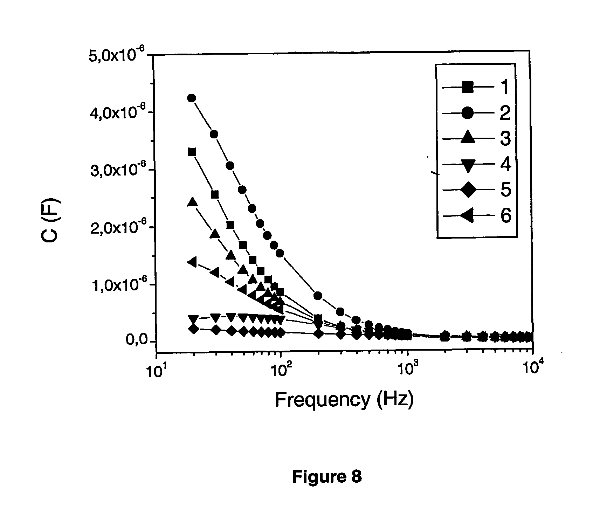

To ascertain the properties of the sensor units of the present invention, electrical measurements of solutions containing a single solute were first made. Figure 8 shows the response of an array of 6 sensor units (as shown in Table 2) for a solution of NaCl 5mM, furnished in terms of capacitance of the microelectrode in function of frequency. The electrical response was obtained through the use of an impedance analyser (mentioned above). Each one of the sensor units can obtain a distinct and characteristic response pattern for the substance to be analysed, which serves as a pattern to characterise the type of taste of the substance.

Equally important as the analysis of merely one substance is the qualitative and quantitative differentiation between various substances, which is possible using the sensory system of the present invention. The number of and the composition of the film of each sensor unit must be optimised in accordance with the characteristics of the beverage to be analysed.

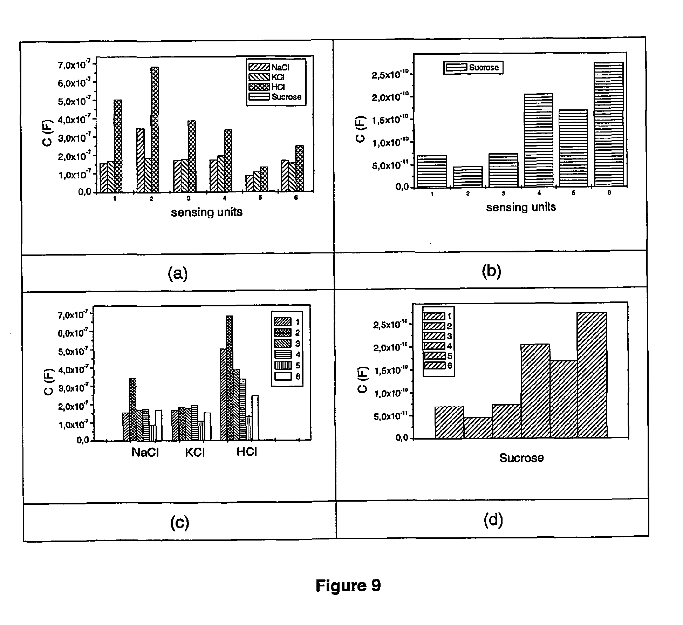

Figure 9 shows that a different and characteristic response pattern is obtained for each substance analysed: with NaCl and KCl representing the salt taste, HCl representing sour and saccharose representing sweet. The results for saccharose - because it does not form an electrolyte - are much lower than the others due to the large difference in response signal intensity compared with other substances which are polar. The solutions analysed were prepared with deionised or ultra-pure water. Therefore, it can be demonstrated that taste analysis with merely one measurement frequency of the electrical response shortens the time of analysis and optimises use in the field.

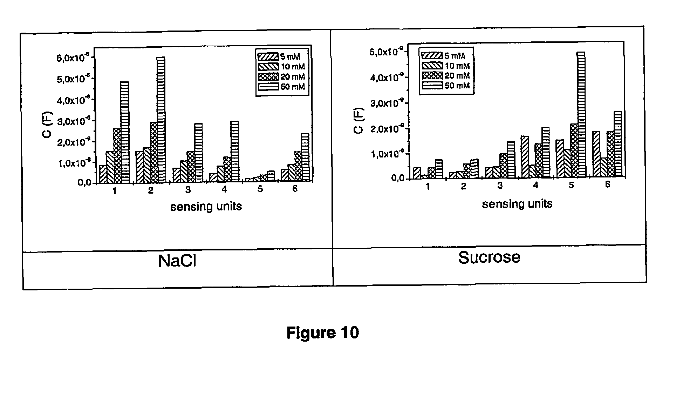

The separation between salt and sweet, in different concentrations, may be done without any problem through the measurement method of the present invention. Figure 10 demonstrates this possibility.

As mentioned above, the quantification can also easily be done using appropriate software such as those existing for the analysis of neural networks. Also, there occurs a differentiated response sensibility for each sensor unit in accordance with the "taste" characteristics of the substance analysed. As an example of this, it can be seen that sensors 4, 5 and 6 show greater sensitivity to sweet taste. As mentioned earlier, the composition of these sensor units can be optimised in accordance with the system to be analysed. The response signal obtained for each sensor unit is proportional to the quantity of the substance present in the liquid phase (aqueous solution). Solutions of NaCl and saccharose in greater concentrations of 100 mM and 300 mM were also measured. The signal distinction between the various sensor units was systematic and characteristic for each one of the solutions analysed and can also be reproduced.

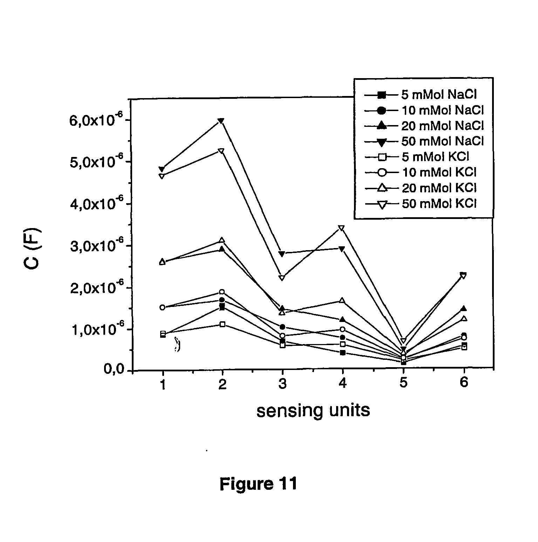

Another interesting point concerning this array of sensors is that it can also be employed to differentiate between two types of salts - such as NaCl from KCl - in different concentrations, including those below the limits of detection by the human organism (5 mM) and those reported in the literature of other inventions. According to C. Pfaffman (Handbook of Physiology, sec. 1, Neurophysiology, Vol. 1, J. Field pub. American Physiological Society, Washington DC - 1959) the minimum concentration of NaCl in an aqueous solution detectable by the human body is of 10 mM. Figure 11 demonstrates this possibility well.

The type of system of the present invention, as well as modifications for adjustments obvious to experts in the matter, can also be employed to evaluate beverages in general. As an example of a practical purpose, the use of this sensory system is demonstrated through the analysis of distinct types of mineral water, chamomile teas and coffees.

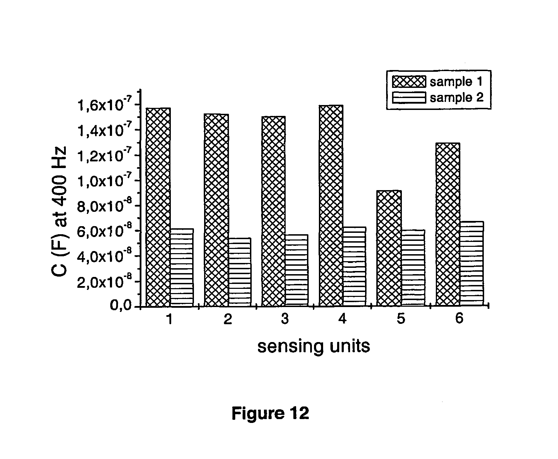

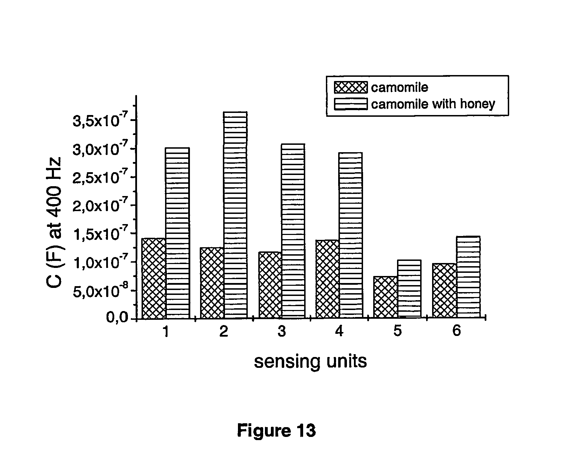

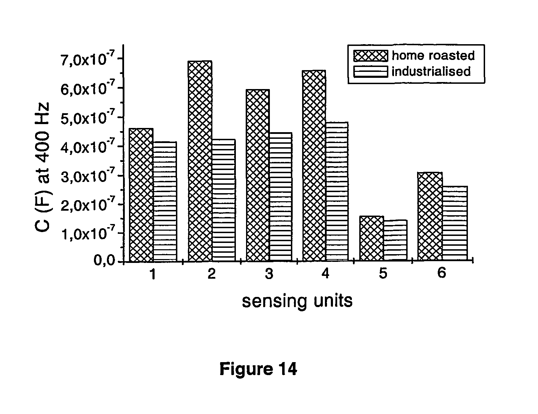

Figures 11, 12 and 13 illustrate the capacitance response signals (in 400 Hz) when the sensors are immersed in the beverages. It can be seen that a very different and characteristic response pattern is obtained for each type of beverage analysed. The information from the mineral water companies corroborate the results obtained since the one that contained the most mineral salts presented a greater intensity of electrical signal response, as shown in Figure 12. Figure 13 demonstrates that the chamomile teas analysed differed only in odour related to whether or not they contained honey (information provided by the manufacturers). Figure 14 shows the difference between two types of coffee, one industrialised and the other commercial. It is important to stress that it was practically impossible to distinguish differences in flavour amongst the samples of water, tea and coffee when analysed by non-specialised persons - which was not the case for the sensors used.

This is a strong indicative of the sensitivity of the response provided by this device, which proved to be more sensitive in differentiating similar substances than the human biological system.

The results obtained in the examples with the use of an array consisting of various different sensor units made of complexing conductive polymers and complexing substances in sensory systems with measurement of (AC) electrical current provide greater sensitivity than the present "electronic tongues" (merely based on lipids and potentiometric readings). The polymers employed in the present inventions may also detect more accurate variations in the case of acid solutions due to the process of doping these materials undergo at certain pH values. Such results allow the manufacture of sensors that are capable of being used not only for qualifying food products but that also can be used in systems for the analysis of cosmetics, pharmaceutics, perfumes etc.

It is also important to highlight that the sensory system of the invention is characterised by having a low-cost and easily reproducible construction.

## Claims

1. Sensor for the analysis of mixtures by global selectivity comprising at least two sensor units consisting of:
(a) interdigitated electrodes or microelectrodes coated with an ultra-fine film of a membrane forming material possessing physicochemical affinity for the substances peculiar to the mixture to be analysed, selected from the group consisting of complexing polymer conductor, complexing substances and combinations thereof and, wherein the ultra-fine film is constituted of 1 to 20 monolayers, with each layer having a thickness varying from 0.5 to 50 nm (5 to 500 Å).

2. Sensor according to claim 1 wherein the sensor further comprises one or more of:
(b) non-coated interdigitated electrodes or microelectrodes.

3. Sensor according to claim 1 wherein the interdigitated electrodes or microelectrodes are of metal selected from the group consisting of gold, platinum, copper or aluminium.

4. Sensor according to claim 1 wherein the complexing polymer conductor is selected from the group consisting of organic polymers possessing poly-conjugated electron- π systems.

5. Sensor according to claim 4 wherein the complexing polymer conductor is selected from the group consisting of polyaniline; polypyrrol; polyacetylene; polydiacetylene; polythiophene; poly(3,4-ethylenedioxithiophene) (PEDOT); polyisothionaphthalene; poly-heteroarylenovinyline, in which the heteroarylene group may be thiophene, furane or pyrrol;
poly-p-phenylene, polyophthalocyanine, as well their derivatives and their mixtures.

6. Sensor according to claim 5 wherein each monolayer has a thickness of around 2 nm (20 Å).

7. Sensor according to claim 5 wherein the ultra-fine film is constituted of around 10 monolayers.

8. Sensor according to claim 5 wherein the maximum thickness of the film is of 50 nm (500 Å).

9. Sensor according to claim 1 wherein the mixture to be analysed is a beverage or liquid food and the complexing substance is selected from the group consisting of lipid compounds, metallic complexes and enzymes.

10. Sensor according to claim 9 wherein the lipid compound is selected from the group consisting of stearic acid, caproic acid and caprylic acid.

11. Sensor according to claim 10 wherein the lipid compound is stearic acid.

12. Sensor according to claim 1 wherein the mixture to be analysed is water from a natural source and the complexing substance is selected from the group consisting of sulphonated lignin, complexing organic acids and complexes containing metallic ions, as well their mixtures.

13. Sensor according to claim 12 wherein the mixture to be analysed is water from a natural source containing humic substances and the complexing substance is selected from the group consisting of sulphonated lignin, complexing organic acids and their mixtures.

14. Sensor according to claim 13 wherein
the complexing organic acid is selected from the group consisting of camphosulphonic acid and toluenesulphonic acid.

15. Sensor according to claim 12 wherein the metallic ion is selected from the group consisting of copper, iron and aluminium.

16. Sensor according to claim 1 or 2, wherein the sensor is a taste sensor for the analysis of liquid systems by global selectivity comprising at least two sensor units consisting of:
(a) interdigitated electrodes or microelectrodes covered with an ultra-fine film of a complexing polymer conductor, optionally combined with at least one complexing substance;
(b) interdigitated electrodes or microelectrodes coated with an ultra-fine film of at least one complexing substance possessing physicochemical affinity for the substances that compose the flavour and, optionally
(c) non-coated interdigitated electrodes or microelectrodes.

17. Taste sensor according to claim 16 wherein the interdigitated electrodes or microelectrodes are of metal selected from the group consisting of gold, platinum, copper or aluminium.

18. Taste sensor according to claim 16 wherein the complexing polymer conductor is an organic polymer possessing poly-conjugated electron-π systems selected from the group consisting of polyaniline; polypyrrol; polyacetylene; polydiacetylene; polythiophene; poly(3,4-ethylenedioxithiophene) (PEDOT); polyisothionaphthalene; poly-heteroarylenevinyline, in which the heteroarylene group may be thiophene, furane or pyrrol; poly-p-phenylene, polyophthalocyanine and similars, as well their derivatives and their mixtures.

19. Taste sensor according to claim 18 wherein the organic polymer is polyaniline and/or polypyrrol.

20. Taste sensor according to claim 16 wherein the complexing substance is a lipid compound selected from the group consisting of stearic acid, caproic acid and caprylic acid.

21. Taste sensor according to claim 20 wherein the lipid compound is stearic acid.

22. Taste sensor according to claim 16 comprising at least six sensor units.

23. Taste sensor according to claim 22 comprising six sensor units consisting of an interdigitated electrode or microelectrode, an interdigitated electrode or microelectrode coated in an ultra-fine film of complexing substance, two interdigitated electrodes or microelectrodes coated in an ultra-fine film of at least one complexing polymer conductor and two interdigitated electrodes or microelectrodes coated in an ultra-fine film of at least one complexing polymer conductor mixed with a complexing substance.

24. Taste sensor according to claim 23 wherein each monolayer has a thickness of around 2 nm (20 Å).

25. Taste sensor according to claim 23 **characterised by** the fact that the ultra-fine film is constituted of around 10 monolayers.

26. Taste sensor according to claim 23 wherein the maximum thickness of the film is of 50 nm (500 Å).

27. Use of a sensor according to claim 1 or 2 for the detection of humic substances within the analysis of liquid systems by global selectivity.

28. Use of a sensor for the detection of humic substances according to 27 wherein the interdigitated electrodes or microelectrodes are of metal selected from the group consisting of gold, platinum, copper or aluminium.

29. Sensor for the detection of humic substances according to claim 16 wherein the complexing polymer conductor is an organic polymer possessing poly-conjugated electron-π systems selected from the group consisting of polyaniline; polypyrrol; polyacetylene; polydiacetylene; polythiophene; poly(3,4-ethylenedioxithiophene) (PEDOT); polyisothionaphthalene; poly-heteroarylenevinyline, in which the heteroarylene group may be thiophene, furane or pyrrol; poly-p-phenylene, polyophthalocyanine and similars, as well their derivatives and their mixtures.

30. Sensor for the detection of humic substances according to claim 18 wherein that the organic polymer is polyaniline and/or poly(o-ethoxyaniline).

31. Use of a sensor for the detection of humic substances according to claim 27 wherein the complexing substance is selected from the group consisting of sulphonated lignin and complexing organic acids.

32. Use of a sensor for the detection of humic substances according to claim 27 wherein the ultra-fine film is obtained by self-mounted alternate layers of polyanion and polycation.

33. Use of a sensor for the detection of humic substances according to claim 32 wherein the polyanion is sulphonated lignin and the polycation is poly(o-ethoxyaniline).

34. Use of a sensor for the detection of humic substances according to claim 27 comprising at least nine sensor units.

35. Use of a sensor for the detection of humic substances according to claim 34 comprising nine sensor units consisting of an interdigitated electrode or microelectrode, three
interdigitated electrodes or microelectrodes coated in an ultra-fine film of at least one complexing polymer conductor, four interdigitated electrodes or microelectrodes coated in an ultra-fine film of at least one complexing polymer conductor mixed with at least one complexing substance and an interdigitated electrode or microelectrode coated in an ultra-fine film of at least one complexing polymer conductor.

36. Use of a sensor for the detection of humic substances according to claim 27 wherein each monolayer has a thickness of around 2 nm (20 Å).

37. Use of a sensor for the detection of humic substances according to claim 27 wherein the ultra-fine film is constituted of around 10 layers.

38. Use of a sensor for the detection of humic substances according to claim 27 wherein the maximum thickness of the film is of 50 nm (500 Å).

39. Sensory system comprising:
(1) at least two sensor units according to claim 1 or 2 generating electric signals,
(2) means of receiving the electrical signal, obtained by means of measuring alternating current, emitted by the sensor units in contact with the material to be analyzed and
(3) means of processing the measurements for the analysis of the response pattern of the array formed by the different sensor units.

## Patentansprüche

1. Sensor für die Analyse von Mischungen durch globale Selektivität umfassend wenigstens zwei Sensoreinheiten bestehend aus:
(a) ineinandergreifende (interdigitated) Elektroden oder Mikroelektroden, beschichtet mit einem ultrafeinen Film aus einem membranbildenden Material, welches eine physikalisch-chemische Affinität für die Substanzen besitzt, welche der zu analysierenden Mischung eigen sind, ausgewählt aus der Gruppe bestehen aus komplexbildendem leitfähigem Polymer, komplexbildenden Substanzen und Kombinationen dieser, und wobei der ultrafeine Film aus 1 bis 20 Monoschichten besteht, wobei jede Schicht eine Dicke aufweist, die zwischen 0,5 bis 50 nm (5 bis 500 A) liegt.

2. Sensor nach Anspruch 1, wobei der Sensor des Weiteren ein oder mehrere umfasst aus:
(b) nicht beschichteten, ineinandergreifenden Elektroden oder Mikroelektroden.

3. Sensor nach Anspruch 1, wobei die ineinandergreifenden Elektroden oder Mikroelektroden aus Metall bestehen, gewählt aus der Gruppe bestehend aus Gold, Platin, Kupfer oder Aluminium.

4. Sensor nach Anspruch 1, wobei das komplexbildende leitfähige Polymer gewählt ist aus der Gruppe bestehend aus organischen Polymeren, welche polykonjugierte Elektronen-π-Systeme besitzen.

5. Sensor nach Anspruch 4, wobei das komplexbildende leitfähige Polymer gewählt ist aus der Gruppe bestehend aus Polyanilin; Polypyrrol; Polyacetylen; Polydiacetylen; Polythiophen; Poly(3,4-Ethylendioxithiophen) (PEDOT); Polyisothionaphthalin; Polyheteroarylenvinylin, wobei die Heteroarylengruppen Thiophen, Furan oder Pyrrol sein können; Poly-p-phenylen, Polyophthalocyanin, wie auch deren Derivate und deren Mischungen.

6. Sensor nach Anspruch 5, wobei jede Monoschicht eine Dicke von ungefähr 2 nm (20 A) aufweist.

7. Sensor nach Anspruch 5, wobei der ultrafeine Film aus ungefähr zehn Monoschichten besteht.

8. Sensor nach Anspruch 5, wobei die maximale Dicke des Films 50 nm (500 Å) beträgt.

9. Sensor nach Anspruch 1, wobei die zu analysierende Mischung ein Getränk oder flüssiges Nahrungsmittel ist, und die komplexbildende Substanz gewählt ist aus der Gruppe bestehend aus Lipidverbindungen, metallischen Komplexen und Enzymen.

10. Sensor nach Anspruch 9, wobei die Lipidverbindung gewählt ist aus der Gruppe bestehend aus Stearinsäure, Capronsäure und Caprylsäure.

11. Sensor nach Anspruch 10, wobei die Lipidverbindung Stearinsäure ist.

12. Sensor nach Anspruch 1, wobei die zu analysierende Mischung Wasser aus einer natürlichen Quelle ist und die komplexbildende Substanz gewählt ist aus der Gruppe bestehend aus sulfoniertem Lignin, komplexbildenden organischen Säuren und Komplexe enthaltend metallische Ionen, wie auch deren Mischungen.

13. Sensor nach Anspruch 12, wobei die zu analysierende Mischung Wasser aus einer natürlichen Quelle, welche Huminstoffe enthält, ist, und die komplexbildende Substanz gewählt ist aus der Gruppe bestehend aus sulfoniertem Lignin, komplexbildenden organischen Säuren und deren Mischungen.

14. Sensor nach Anspruch 13, wobei
die komplexbildende organische Säure gewählt ist aus der Gruppe bestehend aus Camphosulfonsäure und Toluolsulfonsäure.

15. Sensor nach Anspruch 12, wobei das metallische Ion gewählt ist aus der Gruppe bestehend aus Kupfer, Eisen und Aluminium.

16. Sensor nach Anspruch 1 oder 2, wobei der Sensor ein Geschmackssensor zur Analyse von flüssigen Systemen durch globale Selektivität ist, umfassend wenigstens zwei Sensoreinheiten bestehend aus:
(a) ineinandergreifenden Elektroden oder Mikroelektroden, welche mit einem ultrafeinen Film aus einem komplexbildenden leitfähigen Polymer bedeckt sind, gegebenenfalls kombiniert mit wenigstens einer komplexbildenden Substanz;
(b) ineinandergreifenden Elektroden oder Mikroelektroden, beschichtet mit einem ultrafeinen Film aus wenigstens einer komplexbildenden Substanz, welche physikalischchemische Affinität für die Substanzen besitzt, welche den Geschmack ausmachen, und gegebenenfalls
(c) nicht beschichteten ineinandergreifenden Elektroden oder Mikroelektroden.

17. Geschmackssensor nach Anspruch 16, wobei die ineinandergreifenden Elektroden oder Mikroelektroden aus Metall bestehen, gewählt aus der Gruppe bestehend aus Gold, Platin, Kupfer oder Aluminium.

18. Geschmackssensor nach Anspruch 16, wobei dai komplexbildende leitfähige Polymer ein organisches Polymer ist, welches polykonjugierte Elektronen-π-Systeme besitzt, gewählt aus der Gruppe bestehend aus Polyanilin; Polypyrrol; Polyacetylen; Polydiacetylen; Polythiophen; Poly(3,4-ethylendioxithiophen) (PEDOT); Polyisothionaphthalen; Poly-Heteroarylenvinylin, in welchem die Heteroarylengruppen Thiophen, Furan oder Pyrrol sein können; Poly-p-phenylen, Polyophthalocyanin und ähnliche, wie auch ihre Derivate und ihre Mischungen.

19. Geschmackssensor nach Anspruch 18, wobei das organische Polymer Polyanilin und/oder Polypyrrol ist.

20. Geschmackssensor nach Anspruch 16, wobei die komplexbildende Substanz eine Lipidverbindung ist, gewählt aus der Gruppe bestehend aus Stearinsäure, Capronsäure und Caprylsäure.

21. Geschmackssensor nach Anspruch 20, wobei die Lipidverbindung Stearinsäure ist.

22. Geschmackssensor nach Anspruch 16, umfassend wenigstens sechs Sensoreinheiten.

23. Geschmackssensor nach Anspruch 22, umfassend sechs Sensoreinheiten bestehend aus einer ineinandergreifenden Elektrode oder Mikroelektrode, einer ineinandergreifende Elektrode oder Mikroelektrode beschichtet mit einem ultrafeinen Film aus komplexbildender Substanz, zwei ineinandergreifenden Elektroden oder Mikroelektroden beschichtet mit einem ultrafeinen Film aus wenigstens einem komplexbildenden leitfähigem Polymer und zwei ineinandergreifenden Elektroden oder Mikroelektroden beschichtet mit einem ultrafeinen Film aus wenigstens einem komplexbildenden leitfähigen Polymer gemischt mit einer komplexbildenden Substanz.

24. Geschmackssensor nach Anspruch 23, wobei jede Monoschicht eine Dicke von ungefähr 2 nm (20 A) aufweist.

25. Geschmackssensor nach Anspruch 23, **dadurch gekennzeichnet, dass** der ultrafeine Film aus ungefähr zehn Monoschichten besteht.

26. Geschmackssensor nach Anspruch 23, wobei die maximale Dicke des Films 50 nm (500 Å) beträgt.

27. Verwendung eines Sensors nach Anspruch 1 oder 2 zur Ermittlung von Huminsubstanzen in der Analyse flüssiger Systeme durch globale Selektivität.

28. Verwendung eines Sensors zur Ermittlung von Huminstoffen gemäß Anspruch 27, wobei die ineinandergreifenden Elektroden oder Mikroelektroden aus Metall bestehen, gewählt aus der Gruppe bestehend aus Gold, Platin, Kupfer oder Aluminium.

29. Sensor zur Ermittlung von Huminstoffen gemäß Anspruch 16, wobei das komplexbildende leitfähige Polymer ein organisches Polymer ist, welches polykonjugierte Elektronen-π-Systeme besitzt, gewählt aus der Gruppe bestehend aus: Polyanilin; Polypyrrol; Polyacetylen; Polydiacetylen; Polythiophen; Poly(3,4-ethylendioxithiophen) (PEDOT); Polyisothionaphthalin; Poly-Heteroarylenvinylin, in welchem die Heteroarylengruppen Thiophen, Furan oder Pyrrol sein können; Poly-p-phenylen, Polyophthalocyanin und ähnliche, wie auch ihre Derivate und ihre Mischungen.

30. Sensor zur Ermittlung von Huminsubstanzen nach Anspruch 18, wobei das organische Polymer Polyanilin und/oder Poly(o-ethoxyanilin) ist.

31. Verwendung eines Sensors zur Ermittlung von Huminstoffen nach Anspruch 27, wobei die komplexbildende Substanz gewählt ist aus der Gruppe bestehend aus sulfoniertem Lignin oder komplexbildenden organischen Säuren.

32. Verwendung eines Sensors zur Ermittlung von Huminstoffen nach Anspruch 27, wobei der ultrafeine Film erhalten wird durch selbstaufbauende bzw. selbstbefestigende abwechselnde Schichten aus Polyanion und Polykation.

33. Verwendung eines Sensors zur Ermittlung von Huminstoffen nach Anspruch 32, wobei das Polyanion sulfoniertes Lignin ist und das Polykation Poly(o-epoxyanilin).

34. Verwendung eines Sensors zur Ermittlung von Huminstoffen nach Anspruch 27, umfassend wenigstens neun Sensoreinheiten.

35. Verwendung eines Sensors zur Ermittlung von Huminstoffen nach Anspruch 34, umfassend neun Sensoreinheiten bestehend aus einer ineinandergreifenden Elektrode oder Mikroelektroden, drei ineinandergreifenden Elektroden oder Mikroelektroden beschichtet mit einem ultrafeinen Film aus wenigstens einem komplexbildenden leitfähigen Polymer, vier ineinandergreifenden Elektroden oder Mikroelektroden beschichtet mit einem ultrafeinen Film aus wenigstens einem komplexbildenden leitfähigen Polymer, gemischt mit wenigstens einer komplexbildenden Substanz, und einer ineinandergreifenden Elektrode oder Mikroelektrode beschichtet in einem ultrafeinem Film aus wenigstens einem komplexbildenden Polymerleiter.

36. Verwendung eines Sensors zur Ermittlung von Huminstoffen nach Anspruch 27, wobei jede Monoschicht eine Dicke von ungefähr 2 nm (20 Å) aufweist.

37. Verwendung eines Sensors zur Ermittlung von Huminstoffen nach Anspruch 27, wobei der ultrafeine Film aus ungefähr zehn Schichten besteht.

38. Verwendung eines Sensors zur Ermittlung von Huminstoffen nach Anspruch 27, wobei die maximale Dicke des Films 50 nm (500 Å) beträgt.

39. Sensorisches System umfassend:
(1) wenigstens zwei Sensoreinheiten gemäß Anspruch 1 oder 2, welche elektrische Signale erzeugen,
(2) Mittel, um das elektrische Signal zu empfangen, welches mittels Messwechselstrom erhalten wurde, emittiert von den Sensoreinheiten in Kontakt mit dem zu analysierenden Material, und
(3) Mittel zur Verarbeitung der Messungen zur Analyse des Reaktionsmusters des durch die unterschiedlichen Sensoreinheiten gebildeten Feldes.

## Revendications

1. Capteur pour l'analyse de mélanges par la sélectivité globale, comprenant au moins deux unités de capteurs composées :
a) d'électrodes ou de microélectrodes interdigitales revêtues d'un film ultrafin d'un matériau formant membrane possédant une affinité physicochimique pour les substances particulières au mélange à analyser, sélectionnées parmi le groupe constitué par un conducteur polymère complexant, des substances complexantes et des combinaisons de ceux-ci, et dans lequel le film ultrafin est constitué de 1 à 20 monocouches, chaque couche ayant une épaisseur variant de 0,5 à 50 nm (5 à 500 Å).

2. Capteur selon la revendication 1, dans lequel le capteur comprend en outre une ou plusieurs :
b) électrodes ou microélectrodes interdigitales non-revêtues.

3. Capteur selon la revendication 1, dans lequel les électrodes ou microélectrodes interdigitales sont faites en un métal sélectionné parmi le groupe constitué par l'or, le platine, le cuivre ou l'aluminium.

4. Capteur selon la revendication 1, dans lequel le conducteur polymère complexant est sélectionné parmi le groupe constitué par des polymères organiques possédant des systèmes d'électron-π poly-conjugués.

5. Capteur selon la revendication 4, dans lequel le conducteur polymère complexant est sélectionné parmi le groupe constitué par une polyaniline, un polypyrrol, un polyacétylène, un polydiacétylène, un polythiophène, un poly(3,4-éthylènedioxithiophène) (PEDOT), un polyisothionaphthalène, un poly-hétéroarylenovinyline, dans lequel le groupe hétéroarylène peut être un thiophène, un furane ou un pyrrol, un poly-p-phénylène, un polyophthalocyanine, de même que leurs dérivés et leurs mélanges.

6. Capteur selon la revendication 5, dans lequel chaque monocouche a une épaisseur d'environ 2 nm (20 Å).

7. Capteur selon la revendication 5, dans lequel le film ultrafin est constitué d'environ 10 monocouches.

8. Capteur selon la revendication 5, dans lequel l'épaisseur maximale du film est de 50 nm (500 Å).

9. Capteur selon la revendication 1, dans lequel le mélange à analyser est une boisson ou un aliment liquide et la substance complexante est sélectionnée parmi le groupe constitué par des composés de lipides, des complexes métalliques et des enzymes.

10. Capteur selon la revendication 9, dans lequel le composé de lipide est sélectionné parmi le groupe constitué par un acide stéarique, un acide caproïque et un acide caprylique.

11. Capteur selon la revendication 10, dans lequel le composé de lipide est un acide stéarique.

12. Capteur selon la revendication 1, dans lequel le mélange à analyser est une eau provenant d'une source naturelle et la substance complexante est sélectionnée parmi le groupe constitué par une lignine sulfonée, des acides organiques complexants et des complexes contenant des ions métalliques, de même que leurs mélanges.

13. Capteur selon la revendication 12, dans lequel le mélange à analyser est une eau provenant d'une source naturelle contenant des substances humiques et la substance complexante est sélectionnée parmi le groupe constitué par une lignine sulfonée, des acides organiques complexants et leurs mélanges.

14. Capteur selon la revendication 13, dans lequel :
- l'acide organique complexant est sélectionné parmi le groupe constitué par un acide camphosulfonique et un acide toluènosulfonique.

15. Capteur selon la revendication 12, dans lequel l'ion métallique est sélectionné parmi le groupe constitué par le cuivre, le fer et l'aluminium.

16. Capteur selon la revendication 1 ou 2, dans lequel le capteur est un capteur de goût pour l'analyse de systèmes liquides par la sélectivité globale, comprenant au moins deux unités de capteurs composées :
a) d'électrodes ou de microélectrodes interdigitales revêtues d'un film ultrafin d'un conducteur polymère complexant, optionnellement combiné à au moins une substance complexante ;
b) d'électrodes ou de microélectrodes interdigitales revêtues d'un film ultrafin d'au moins une substance complexante possédant une affinité physicochimique pour les substances qui composent la saveur ; et optionnellement
c) d'électrodes ou microélectrodes interdigitales non-revêtues.

17. Capteur de goût selon la revendication 16, dans lequel les électrodes ou microélectrodes interdigitales sont faites en un métal sélectionné parmi le groupe constitué par l'or, le platine, le cuivre ou l'aluminium.

18. Capteur de goût selon la revendication 16, dans lequel le conducteur polymère complexant est un polymère organique possédant des systèmes d'électron-π poly-conjugués, sélectionné parmi le groupe constitué par une polyaniline, un polypyrrol, un polyacétylène, un polydiacétylène, un polythiophène, un poly(3,4-éthylènedioxithiophène) (PEDOT), un polyisothionaphthalène, un poly-hétéroarylenovinyline, dans lequel le groupe hétéroarylène peut être un thiophène, un furane ou un pyrrol, un poly-p-phénylène, un polyophthalocyanine ou autre similaire, de même que leurs dérivés et leurs mélanges.

19. Capteur de goût selon la revendication 18, dans lequel le polymère organique est une polyaniline et/ou un polypyrrol.

20. Capteur de goût selon la revendication 16, dans lequel la substance complexante est un composé de lipide sélectionné parmi le groupe constitué par un acide stéarique, un acide caproïque et un acide caprylique.

21. Capteur de goût selon la revendication 20, dans lequel le composé de lipide est un acide stéarique.

22. Capteur de goût selon la revendication 16, comprenant au moins six unités de capteurs.

23. Capteur de goût selon la revendication 22, comprenant six unités de capteurs composées d'une électrode ou microélectrode interdigitale, d'une électrode ou microélectrode interdigitale revêtue d'un film ultrafin d'une substance complexante, de deux électrodes ou microélectrodes interdigitales revêtues d'un film ultrafin d'au moins un conducteur polymère complexant et de deux électrodes ou microélectrodes interdigitales revêtues d'un film ultrafin d'au moins un conducteur polymère complexant mélangé avec une substance complexante.

24. Capteur de goût selon la revendication 23, dans lequel chaque monocouche a une épaisseur d'environ 2 nm (20 Å).

25. Capteur de goût selon la revendication 23, **caractérisé par le fait que** le film ultrafin est constitué d'environ 10 monocouches.

26. Capteur de goût selon la revendication 23, dans lequel l'épaisseur maximale du film est de 50 nm (500 Å).

27. Utilisation d'un capteur selon la revendication 1 ou 2 pour la détection de substances humiques au cours de l'analyse de systèmes liquides par la sélectivité globale.

28. Utilisation d'un capteur pour la détection de substances humiques selon la revendication 27, pour laquelle les électrodes ou microélectrodes interdigitales sont
faites en un métal sélectionné parmi le groupe constitué par l'or, le platine, le cuivre ou l'aluminium.

29. Capteur pour la détection de substances humiques selon la revendication 16, dans lequel le conducteur polymère complexant est un polymère organique possédant des systèmes d'électron-π poly-conjugués, sélectionné parmi le groupe constitué par une polyaniline, un polypyrrol, un polyacétylène, un polydiacétylène, un polythiophène, un poly(3,4-éthylènedioxithiophène) (PEDOT), un polyisothionaphthalène, un poly-hétéroarylenovinyline, dans lequel le groupe hétéroarylène peut être un thiophène, un furane ou un pyrrol, un poly-p-phénylène, un polyophthalocyanine ou autre similaire, de même que leurs dérivés et leurs mélanges.

30. Capteur pour la détection de substances humiques selon la revendication 18, dans lequel le polymère organique est une polyaniline et/ou un poly (o-éthoxyaniline).

31. Utilisation d'un capteur pour la détection de substances humiques selon la revendication 27, pour laquelle la substance complexante est sélectionnée parmi le groupe constitué par une lignine sulfonée et des acides organiques complexants.

32. Utilisation d'un capteur pour la détection de substances humiques selon la revendication 27, pour laquelle le film ultrafin est obtenu par des couches alternées auto-montées d'un poly-anion et d'un poly-cation.

33. Utilisation d'un capteur pour la détection de substances humiques selon la revendication 32, pour laquelle le poly-anion est une lignine sulfonée et le polycation est un poly (o-éthoxyaniline).

34. Utilisation d'un capteur pour la détection de substances humiques selon la revendication 27, le capteur comprenant au moins neuf unités de capteurs.

35. Utilisation d'un capteur pour la détection de substances humiques selon la revendication 34, le capteur comprenant neuf unités de capteurs composées d'une électrode ou microélectrode interdigitale, de trois électrodes ou microélectrodes interdigitales revêtues d'un film ultrafin d'au moins un conducteur polymère complexant, de quatre électrodes ou microélectrodes interdigitales revêtues d'un film ultrafin d'au moins un conducteur polymère complexant mélangé avec au moins une substance complexante et d'une électrode ou microélectrode interdigitale revêtue d'un film ultrafin d'au moins un conducteur polymère complexant.

36. Utilisation d'un capteur pour la détection de substances humiques selon la revendication 27, pour laquelle chaque monocouche a une épaisseur d'environ 2 nm (20 Å).

37. Utilisation d'un capteur pour la détection de substances humiques selon la revendication 27, pour laquelle le film ultrafin est constitué d'environ 10 couches.

38. Utilisation d'un capteur pour la détection de substances humiques selon la revendication 27, pour laquelle l'épaisseur maximale du film est de 50 nm (500 Å).

39. Système sensoriel comprenant :
1) au moins deux unités de capteurs selon la revendication 1 ou 2, générant des signaux électriques ;
2) des moyens de réception du signal électrique, obtenu par des moyens de mesure d'un courant alternatif, émis par les unités de capteurs au contact de la matière à analyser ; et
3) des moyens de traitement des mesures pour l'analyse du motif de réponse du réseau formé par les différentes unités de capteurs.
